# EUROPÄISCHE PATENTSCHRIFT

(11) **EP 3 978 223 B1**
(45) Veröffentlichungstag und Bekanntmachung des Hinweises auf die Patenterteilung: **24.04.2024**
(21) Anmeldenummer: 21195857.4
(22) Anmeldetag: 09.09.2021
(51) Int. Cl.: B29C 49/42, B29C 49/46, B29C 49/06, B29K 67/00, B29L 31/00

(54) **VORRICHTUNG UND VERFAHREN ZUM BEHANDELN VON BEHÄLTNISSEN MIT BEAUFSCHLAGUNG DURCH IONISIERTE LUFT**
DEVICE AND METHOD FOR TREATING CONTAINERS WITH EXPOSURE TO IONIZED AIR
DISPOSITIF ET PROCÉDÉ DE TRAITEMENT DES RÉCIPIENTS PAR EXPOSITION À AIR IONISÉ

(30) Priorität: 30.09.2020 DE 102020125478
(43) Veröffentlichungstag der Anmeldung: 06.04.2022
(73) Patentinhaber: Krones AG, 93073 Neutraubling (DE)
(72) Erfinder: Merl, Ulrich, 93073 Neutraubling (DE)
(74) Vertreter: Hannke Bittner & Partner mbB Regensburg

(56) Entgegenhaltungen:
- DE-A1- 10 140 906
- DE-A1-102011 107 772
- US-A1- 2018 009 646

## Beschreibung

Die vorliegende Erfindung bezieht sich auf eine Vorrichtung und ein Verfahren zum Behandeln von Behältnissen und insbesondere von Kunststoffvorformlingen. Gattungsgemäße Vorrichtungen und Verfahren sind in den Patentschriften US2018/009646A1, DE10-2011-107772A1 und DE101-40906A1 beschrieben.

In der getränkeherstellenden Industrie ist es seit langem bekannt, Kunststoffvorformlinge zu erwärmen und anschließend beispielsweise mittels einer Streckblasmaschine zu einem Behältnis wie etwa einer Plastikflasche umzuformen. Dabei ist es aus dem internen Stand der Technik der Anmelderin bekannt, derartige Kunststoffvorformlinge beispielsweise auch vor deren Erwärmung mit einem fließfähigen Medium und insbesondere mit gefilterter ionisierter Luft zu beaufschlagen bzw. auszublasen, um diese von innen zu reinigen.

Diese Vorgehensweise erlaubt zwar das Reinigen der Kunststoffvorformlinge, nicht jedoch das Abtöten von mikrobiologischen Keimen, die sich insbesondere auch im Inneren dieser Kunststoffvorformlinge befinden können.

Der vorliegenden Erfindung liegt daher die Aufgabe zugrunde, in insbesondere prozessökonomischer Weise auch ein Abtöten von derartigen Verkeimungen in Behältnissen und insbesondere auch in Kunststoffvorformlingen zu ermöglichen. Diese Aufgabe wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln von Kunststoffvorformlingen weist eine Transporteinrichtung auf, welche die Kunststoffvorformlinge entlang eines vorgegebenen Transportpfads transportiert sowie eine erste Beaufschlagungseinrichtung, welche die Kunststoffvorformlinge, während deren Transport entlang eines ersten Transportpfadabschnitts dieses Transportpfads, durch deren Mündung hindurch mit einem ersten fließfähigen Medium beaufschlagt.

Erfindungsgemäß weist die Vorrichtung eine zweite Beaufschlagungseinrichtung auf, welche die Kunststoffvorformlinge während deren Transport entlang eines zweiten Transportpfadabschnitts (insbesondere des oben genannten Transportpfads) durch deren Mündung hindurch mit einem zweiten fließfähigen Medium beaufschlagt. Es wird daher im Rahmen der Erfindung vorgeschlagen, dass die Kunststoffvorformlinge während ihres Transports mit der Vorrichtung mit den zwei - insbesondere unterschiedlichen - fließfähigen Medien beaufschlagt werden. Bei den fließfähigen Medien kann es sich sowohl um gasförmige Medien handeln wie etwa (Druck)luft als auch um flüssige Medien wie Flüssigkeiten oder auch dampfförmige Medien oder dergleichen.

Besonders bevorzugt sind die Transportpfadabschnitte vollständig voneinander getrennt, das heißt, sie überschneiden sich nicht. Bevorzugt schließt sich der zweite Transportpfadabschnitt im Wesentlichen unmittelbar an den ersten Transportpfadabschnitt an.

Bei einer besonders bevorzugten Ausführungsform weist die Transporteinrichtung einen beweglichen und insbesondere einen drehbaren Träger auf, an dem eine Vielzahl von Halteeinrichtungen zum Halten der Kunststoffvorformlinge angeordnet sind. Dabei sind besonders bevorzugt diese Halteeinrichtungen vereinzelt angeordnet, sodass auch die Kunststoffvorformlinge vereinzelt transportiert werden können. Bevorzugt werden die Kunststoffvorformlinge mit einer vorgegebenen und insbesondere konstanten Teilung bezüglich einander transportiert.

Bei einer bevorzugten Ausführungsform ist jeder dieser Halteeinrichtungen wenigstens eine und bevorzugt genau eine Beaufschlagungseinrichtung zugeordnet.

Bei einer weiteren bevorzugten Ausführungsform weist die Beaufschlagungseinrichtung eine Zurverfügungstellungseinheit auf, welche Druckluft und insbesondere sterile Druckluft zur Verfügung stellt. So kann beispielsweise eine Kompressoreinrichtung vorgesehen sein, welche Druckluft erzeugt.

Besonders bevorzugt weist die erste Zurverfügungstellungseinheit auch Filtereinrichtungen insbesondere Sterilfilter bzw. Reinigungseinrichtungen zum Sterilisieren der Luft auf. So können beispielsweise HEPA - Filter vorgesehen sein.

Besonders bevorzugt ist eine Kompressoreinrichtung vorgesehen, welche Druckluft erzeugt und besonders bevorzugt ist diese Kompressoreinrichtung stationär angeordnet. Bei dieser Ausgestaltung wird das unter Druck stehende Medium, beispielsweise Druckluft, über einen Drehverteiler auf wenigstens eine Beaufschlagungseinrichtung und bevorzugt auf eine Vielzahl von Beaufschlagungseinrichtungen aufgeteilt.

Bei einer weiteren bevorzugten Ausführungsform weist wenigstens eine Beaufschlagungseinrichtung und/oder eine zur Verfügungsstellungseinheit eine lonisiereinrichtung zum lonisieren von Luft auf. Auf diese Weise kann den einzelnen Kunststoffvorformlingen ionisierte Sterilluft und insbesondere ionisierte und sterile Druckluft zur Verfügung gestellt werden.

Bei einer weiteren vorteilhaften Ausführungsform weist wenigstens eine Beaufschlagungseinrichtung eine Zurverfügungsstellungseinheit auf, welche ein fließfähiges Sterilisationsmedium zur Verfügung stellt. Dabei kann es sich insbesondere, aber nicht ausschließlich um Wasserstoffperoxid (H₂O₂) handeln. Es wäre jedoch auch etwa die Verwendung von Peressigsäure denkbar.

Bei einer weiteren bevorzugten Ausführungsform weisen die erste und die zweite Beaufschlagungseinrichtung ein Beaufschlagungselement auf, welches das fließfähige Medien in die Kunststoffvorformlinge einführt (bzw. welche die fließfähigen Medien in die Kunststoffvorformlinge einführen) . Dabei kann es sich beispielsweise um eine Düse oder dergleichen handeln, mittels derer die jeweilige Substanz in die Preforms bzw. Kunststoffvorformlinge eingeführt wird. Dabei ist bevorzugt dieses Beaufschlagungselement auch auf die Kunststoffvorformlinge zustellbar bzw. in dieselben einführbar.

Bei der Beaufschlagungseinrichtung handelt es sich damit um die gesamte Einheit, welche zum Erzeugen und auch zum Zuführen des jeweiligen Mediums zu den Kunststoffvorformlingen dient. Unter dem Beaufschlagungselement wird dasjenige Element verstanden, welches das jeweilige Medium letztlich den Kunststoffvorformlingen zuführt, wie etwa eine Düse, welche in die Mündungen der Kunststoffvorformlinge hineinragt.

Bei einer bevorzugten Ausführungsform nutzen die erste Beaufschlagungseinrichtung und die zweite Beaufschlagungseinrichtung das gleiche Beaufschlagungselement, um die Kunststoffvorformlinge mit dem jeweiligen fließfähigen Medium zu beaufschlagen. In diesem Falle wird daher die erste Substanz, beispielsweise Wasserstoffperoxid, und auch die zweite Substanz, beispielsweise sterile Druckluft über die gleiche Düse bzw. das gleiche Beaufschlagungselement in die Kunststoffvorformlinge eingegeben.

Bei einer weiteren vorteilhaften Ausführungsform weist die Vorrichtung eine Absaugeinrichtung auf, um ein fließfähiges Medium (wie etwa Luft) aus dem Kunststoffvorformling abzusaugen. Dabei kann beispielsweise eine Substanz, welche Verschmutzungen enthält aus dem Kunststoffvorformling abgesaugt werden. Diese Absaugeinrichtung kann dabei ein Bestandteil der ersten Beaufschlagungseinrichtung sein. So ist es möglich, dass die Beaufschlagung mit dem ersten fließfähigen Medium und die Absaugung bezüglich eines bestimmten Kunststoffvorformlings zu gleichen Zeiträumen erfolgen.

Besonders bevorzugt ist die Vorrichtung derart aufgebaut, dass zunächst eine Absaugung erfolgt, und anschließend eine Beaufschlagung mit Druckluft und/oder mit einem Sterilisationsmedium wie Wasserstoffperoxid.

Bei einer weiteren vorteilhaften Ausführungsform weist die Vorrichtung eine Umschalteinrichtung auf, welche ein Umschalten von einer Beaufschlagung der Kunststoffvorformlinge mit dem ersten fließfähigen Medium mittels der Beaufschlagungselemente zur einer Beaufschlagung der Kunststoffvorformlinge mit dem zweiten fließfähigen Medium mittels der Beaufschlagungselemente umschaltet.

Dabei ist es möglich, dass in Abhängigkeit von einer bestimmten Position der jeweiligen Beaufschlagungseinrichtung bzw. des Beaufschlagungselements entlang der Transporteinrichtung die Beaufschlagung mit dem einen oder dem anderen Medium erfolgt. Damit kann die Beaufschlagung der Kunststoffvorformlinge mit den jeweiligen Medien auch von einer Drehstellung der Transporteinrichtung beispielsweise eines drehbaren Trägers abhängen.

Bevorzugt weist die Umschalteinrichtung eine Vielzahl von Ventilen auf, welche das Umschalten von der Beaufschlagung mit dem ersten fließfähigen Medium auf eine Beaufschlagung mit dem zweiten fließfähigen Medium ermöglichen. Dabei kann jedem Beaufschlagungselement eine derartige Umschalteinrichtung zugeordnet sein. Auf diese Weise lassen sich die einzelnen Beaufschlagungselemente individuell umschalten.

Bei einer weiteren bevorzugten Ausführungsform weist die Vorrichtung eine Verteilereinrichtung auf, um das erste fließfähige Medium oder das zweite fließfähige Medium auf die einzelnen Beaufschlagungseinrichtungen und/oder Beaufschlagungselemente zu verteilen. Dabei kann sowohl für das erste fließfähige Medium als auch für das zweite fließfähige Medium eine derartige Verteileinrichtung zur Verfügung stehen. Besonders bevorzugt handelt es sich bei der Verteileinrichtung um einen sogenannten Drehverteiler.

Weiterhin ist es möglich, dass ein Reservoir für die ersten und zweiten fließfähigen Medien stationär angeordnet ist, und die jeweiligen fließfähigen Medien (beispielsweise über Drehverteiler) auf die Beaufschlagungselemente und letztlich auf die Vorformlinge übertragen werden.

Bei einer weiteren bevorzugten Ausführungsform weist die Transporteinrichtung einen umlaufenden und insbesondere einen bezüglich einer Drehachse drehbaren Träger auf, an dem eine Vielzahl von Halteeinrichtungen zum Halten der Kunststoffvorformlinge angeordnet ist, wobei jeder Halteeinrichtung wenigstens ein und bevorzugt genau ein Beaufschlagungselement zum Beaufschlagen der Kunststoffvorformlinge mit den fließfähigen Medien zugeordnet ist. Bevorzugt sind dabei diese Beaufschlagungselemente oberhalb des drehbaren Trägers angeordnet.

Besonders bevorzugt ist jeder Halteeinrichtung genau eine derartige Beaufschlagungseinrichtung bzw. ein derartiges Beaufschlagungselement zugeordnet. Vorteilhaft ist dieses Beaufschlagungselement in einer Längsrichtung der Kunststoffvorformlinge auf die Kunststoffvorformlinge zustellbar.

Bei einer weiteren bevorzugten Ausführungsform handelt es sich bei der Transporteinrichtung um einen sogenannten Eintaktstern oder eine Eintakteinrichtung, welche die in einer Reihe zugeführten Kunststoffvorformlinge auf eine vorgegebene Teilung bringt. Dabei ist es möglich, dass die Kunststoffvorformlinge mittels einer weiteren Zuführeinrichtung, etwa einer Preformrinne, zugeführt werden. Die Transporteinrichtung weist besonders bevorzugt an ihrem Außenumfang Ausnehmungen auf, welche zum Halten der Kunststoffvorformlinge dienen. Besonders bevorzugt werden dabei die Kunststoffvorformlinge zwischen diesen Ausnehmungen und einer Führungsschiene, die außerhalb der Transporteinrichtung verläuft, gehalten. Damit stellen diese Ausnehmungen die jeweiligen Halteeinrichtungen dar.

Bei einer weiteren bevorzugten Ausführungsform sind die Beaufschlagungselemente gegenüber den Kunststoffvorformlingen auch in einer radialen Richtung des umlaufenden Trägers bewegbar und insbesondere rückstellbar. Auf diese Weise kann auch eine Übergabe der Kunststoffvorformlinge an eine weitere Vorrichtung, wie etwa einem Preform-Ofen, ermöglicht werden.

Die vorliegende Erfindung ist weiterhin auf eine Anordnung zur Behandlung von Kunststoffvorformlingen mit einer Vorrichtung der oben beschriebenen Art gerichtet. Weiterhin weist die Anordnung eine in der Transporteinrichtung der Kunststoffvorformlinge der Vorrichtung nachgeordnete Vorrichtung zum Sterilisieren von Kunststoffvorformlingen oder Kunststoffbehältnissen auf.

Bei einer weiteren bevorzugten Ausführungsform weist die Anordnung einen Reinraum auf, innerhalb dessen wenigstens ein Behandlungsschritt erfolgt, beispielsweise eine Umformung von Kunststoffvorformlingen zu Kunststoffbehältnissen. Besonders bevorzugt findet innerhalb dieses Reinraums zumindest das weitere Sterilisieren von Kunststoffvorformlingen oder Kunststoffbehältnissen mit der besagten nachgeordneten Vorrichtung statt.

Die hier beschriebene Vorrichtung ist besonders bevorzugt außerhalb dieses Reinraumes angeordnet und insbesondere in einem sogenannten Grauraum, in dem ein gewisser Reinigungsgrad vorliegt, der aber noch nicht die für den Reinraum geltenden Reinheitsgrade erreicht.

Dabei sind bevorzugt Schleuseneinrichtungen vorgesehen, um den oder die Kunststoffvorformlinge dem Reinraum zuzuführen. Bei einer weiteren bevorzugten Ausführungsform weist der Reinraum diesen Reinraum begrenzende Wandungen auf, welche den Reinraum gegenüber einer (unsterilen) Umgebung abgrenzen. Dabei ist es möglich, dass ein Teil dieser Wandungen gegenüber einem weiteren Teil dieser Wandungen beweglich ist.

Bei einer weiteren bevorzugten Ausführungsform weist die Anordnung auch eine Erwärmungseinrichtung zum Erwärmen von Kunststoffvorformlingen auf. Auch diese Erwärmungseinrichtung ist dabei bevorzugt der hier beschriebenen Vorrichtung in der Transportrichtung der Kunststoffvorformlinge nachgeordnet. Bei dieser Erwärmungseinrichtung kann es sich beispielsweise um einen Infrarot- oder Mikrowellenofen handeln, in dem die Kunststoffvorformlinge erwärmt werden.

Bei einer weiteren bevorzugten Ausführungsform setzt die oben erwähnte Sterilisationseinrichtung zum Sterilisieren der Kunststoffvorformlinge ein fließfähiges Medium ein. Insbesondere handelt es sich dabei um das gleiche Sterilisationsmedium, welches auch die hier beschriebene Vorrichtung einsetzt. So kann insbesondere ein Sterilisationsmittel wie etwa H₂O₂ über eine Verbindungsleitung von einem einheitlichen Verdampfer sowohl zu der weiteren Vorrichtung als auch zu der hier beschriebenen Vorrichtung geführt werden. Dabei kann auch eine Luftdrehdurchführung vorgesehen sein, wie oben beschrieben. Bevorzugt weist daher die Sterilisationseinrichtung einen Verdampfer zum Erzeugen von Wasserstoffperoxid auf.

Besonders bevorzugt handelt es sich bei der weiteren Vorrichtung zum Sterilisieren von Behältnissen um eine Vorrichtung, welche die bereits gefertigten Behältnisse sterilisiert.

Die vorliegende Erfindung ist weiterhin auf ein Verfahren zum Behandeln von Kunststoffvorformlingen gerichtet, wobei eine Transporteinrichtung die Kunststoffvorformlinge entlang eines vorgegebenen Transportpfads transportiert und eine erste Beaufschlagungseinrichtung die Kunststoffvorformlinge während deren Transport entlang eines ersten Transportpfadabschnitts durch deren Mündung hindurch mit einem ersten fließfähigen Medium beaufschlagt.

Erfindungsgemäß beaufschlagt eine zweite Beaufschlagungseinrichtung die Kunststoffvorformlinge während deren Transports entlang des zweiten Transportpfadabschnitts durch deren Mündung hindurch mit einem zweiten fließfähigen Medium. Besonders bevorzugt handelt es sich bei dem ersten fließfähigen Medium um ionisierte Luft und insbesondere sterile ionisierte Luft und insbesondere ionisierte sterile Druckluft. Besonders bevorzugt bei dem zweiten fließfähigen Medium um ein Sterilisationsmedium und insbesondere um Wasserstoffperoxid (H₂O₂). Es wäre jedoch auch möglich, dass die Kunststoffvorformlinge zuerst mit Wasserstoffperoxid und anschließend mit Sterilluft beaufschlagt werden.

Besonders bevorzugt weist wenigstens eine Beaufschlagungseinrichtung eine Ionisierungseinrichtung zum Ionisieren von Luft auf und/oder wenigstens eine Beaufschlagungseinrichtung ionisiert Luft.

Besonders bevorzugt erfolgt die Beaufschlagung des Kunststoffvorformlings mit dem ersten oder dem zweiten fließfähigen Medium während eines Transports der Kunststoffvorformlinge und insbesondere während eines Transports der Kunststoffvorformlinge entlang kreisförmigen Transportpfads. Es wäre jedoch auch ein getakteter Transport denkbar.

Bei einem weiteren bevorzugten Verfahren erfolgt auch wenigstens zeitweise eine Absaugung eines Mediums und insbesondere Luft aus den Kunststoffvorformlingen. Besonders bevorzugt erfolgt diese Absaugung vor der Beaufschlagung mit dem ersten fließfähigen Medium.

Bei einem weiteren bevorzugten Verfahren werden die Kunststoffvorformlinge in einem weiteren Verfahrensschritt erneut sterilisiert. Dies erfolgt besonders bevorzugt nochmals mit Wasserstoffperoxid. Bei einem weiteren bevorzugten Verfahren werden die Kunststoffvorformlinge erwärmt, und insbesondere mittels eines Infrarotofens erwärmt.

Bei einem weiteren bevorzugten Verfahren werden die Kunststoffvorformlinge von der hier beschriebenen Transporteinrichtung vereinzelt, das heißt, sie werden in Transportrichtung zueinander beabstandet.

Im Rahmen der Erfindung wird daher auch vorgeschlagen, dass ein hier beschriebenes Rinsen bzw. Spülen der Kunststoffvorformlinge auch in einen Sterilisationsprozess einer weiteren Einrichtung, insbesondere eines Füllers eingebunden wird. Dabei ist besonders bevorzugt eine Verbindungsleitung vorgesehen, welche von einem Wasserstoffperoxid-Verdampfer zu einer Luftdrehdurchführung des hier beschriebenen Rinsers der Vorrichtung verläuft.

Bevorzugt wird, wie oben erwähnt, ein zweiter Steuerkreis für das Sterilisationsmittel etwa ein H₂O₂-Gas aufgebaut. Weiterhin wird bevorzugt auch eine Absaugleitung von der AHU (Air Handlung Unit) zu der hier beschriebenen Vorrichtung verlegt. Bei dieser AHU handelt es sich insbesondere um eine Einrichtung welche Sterilluft erzeugt.

Einem bevorzugten Verfahren wird der Kunststoffvorformling zunächst über einen Einlaufstern, wie einen Eintaktstern, in die hier beschriebene Vorrichtung bzw. den Behandlungsstern transportiert. Hier taucht zunächst eine Düse in den Kunststoffvorformling ein und die Absaugung wird aktiviert.

Anschließend wird der Kunststoffvorformling mit ionisierter Sterilluft mit einem vorgegebenen Druck und insbesondere einem Druck, der höher ist als 2,0 bar, bevorzugt höher als 2,5 bar und bevorzugt höher als 3,0 bar gereinigt. Besonders bevorzugt ist der Druck geringer als 10,0 bar, bevorzugt geringer als 8,0 bar, besonders geringer als 6,0 bar, bevorzugt geringer als 4,0 bar und bevorzugt geringer als 3,5 bar.

Diese Reinigung kann dabei über einen vorgegebenen Bereich stattfinden, beispielsweise einen Bereich von 12 Grob-Takten. Zeitleich gleichzeitig kann mit dieser Beaufschlagung auch eine Absaugung erfolgen. Bei einem bevorzugten Verfahren findet also die Beaufschlagung mit der Sterilluft zeitgleich mit einem Absaugen statt. Auf diese Weise kann Schmutz aus den Kunststoffvorformlingen ausgeblasen und abgesaugt werden.

Anschließend wird die Sterilluft abgeschaltet und - bevorzugt über einen zweiten Steuerkreis ein Umschaltventil, oben als Umschalteinrichtung bezeichnet - betätigt. Dieses Umschaltventil beaufschlagt H₂O₂-Gas von einem Verdampfer eines Sterilisator-Ventilknotens in den Kunststoffvorformling und nimmt auf diese Weise eine Vorbehandlung in diesem vor. Auf diese Weise kann die Effektivität und Produktionssicherheit der eigentlichen Sterilisation im Behandlungsmodul oder im Sterilisator verbessert werden.

Durch die hier beschriebene Erfindung erfolgt damit eine Reinigung und Vorbehandlung von Kunststoffvorformlingen insbesondere in einem Transportstern. Weiterhin wird die Entkeimungsleistung der gesamten Anordnung verbessert. Daneben wird auch eine höhere Produktionssicherheit durch eine Vorbehandlung erreicht.

Andererseits sind gegenüber dem Stand der Technik nur geringfügige Modifikationen an bereits vorhandener Technik nötig.

Weitere Vorteile und Ausführungsformen ergeben sich aus den beigefügten Zeichnungen. Darin zeigen:
- Fig. 1: eine Darstellung einer Anordnung zum Herstellen von Behältnissen;
- Fig. 2: eine schematische Darstellung einer erfindungsgemäßen Vorrichtung; und
- Fig. 3: eine weitere Ansicht einer erfindungsgemäßen Vorrichtung.

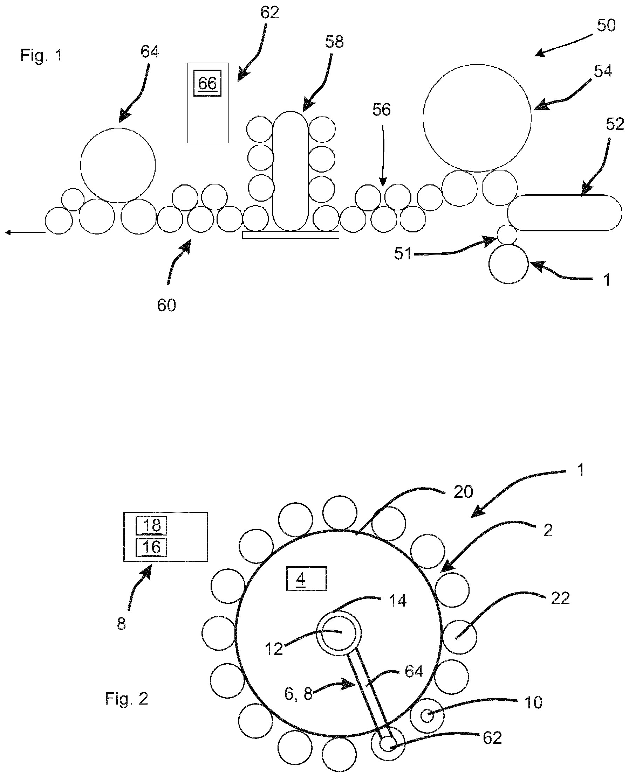

Figur 1 zeigt eine Darstellung einer Anordnung 50 zum Herstellen von Behältnissen. Diese weist einen Ofen 52 auf, in dem Kunststoffvorformlinge erwärmt werden. An diesen Ofen schließt sich eine Umformungseinrichtung 54 an, welche die erwärmten Behältnisse zu Kunststoffflaschen umformt. An diese Umformungseinrichtung 54, bei der es sich beispielsweise um eine Streckblasmaschine handeln kann, schließt sich eine optionale erste Sterilisationsvorrichtung 56 an, welche die Behältnisse sterilisiert.

Das Bezugszeichen 58 kennzeichnet eine (optional vorhandene) Beschichtungseinrichtung zum Beschichten der gefertigten Behältnisse.

Das Bezugszeichen 60 kennzeichnet eine weitere Sterilisationsanordnung zum Sterilisieren der Behältnisse. Diese weist auch eine Zurverfügungstellungseinrichtung 62 auf, welche anstelle des Sterilisationsmediums wie etwa Wasserstoffperoxidgas zur Verfügung stellt. Das Bezugszeichen 66 kennzeichnet einen Verdampfer, der Bestandteil dieser Zurverfügungstellungseinrichtung ist und der Wasserstoffperoxid-Dampf herstellt. Dieser Wasserstoffperoxid-Dampf wird einerseits für die weitere Sterilisationseinrichtung 60 verwendet und auch für die oben beschriebene Vorrichtung.

Das Bezugszeichen 64 kennzeichnet eine Füllereinrichtung, welche die gefertigten Behältnisse mit einer Flüssigkeit, wie einem Getränk, befüllt. An diese Füllereinrichtung schließt sich eine Verschließereinrichtung an, welche die Behältnisse mit Verschlüssen verschließt.

Dabei kann auch eine Sterilisationseinrichtung zum Sterilisieren dieser Verschlüsse vorgesehen sein.

Das Bezugszeichen 1 bezieht sich auf die hier beschriebene erfindungsgemäße Vorrichtung, welche zum Spülen und Vorsterilisieren der Kunststoffvorformlinge dient. An diese Vorrichtung schließt sich eine Zuführeinrichtung an, welche die Kunststoffvorformlinge dem Ofen 52 zuführt.

Figur 2 zeigt eine schematische Darstellung der erfindungsgemäßen Vorrichtung 1. Diese weist eine Transporteinrichtung 2 auf, wie etwa ein Trägerrad 20, an dem eine Vielzahl von Behandlungsstationen angeordnet ist. Diese Stationen weisen jeweils Halteeinrichtungen 22 zum Halten von Kunststoffvorformlingen 10 auf.

Die Bezugszeichen 6, 8 beziehen sich schematisch auf Beaufschlagungseinrichtungen, welche dazu dienen, die Kunststoffvorformlinge 10 mittels eines Beaufschlagungselements 62 mit einem fließfähigen Medium zu beaufschlagen. Zu diesem Zweck sind auch Verbindungsleitungen 64 vorgesehen, welche von einer Verteilereinrichtung, beispielsweise Drehverteilern 12, 14 zu dem Beaufschlagungselement führen. Dabei dient das Beaufschlagungselement 62 sowohl zur Beaufschlagung der Kunststoffvorformlinge mit dem ersten fließfähigen Medium als auch zur Beaufschlagung der Kunststoffvorformlinge mit dem zweiten fließfähigen Medium.

Das Bezugszeichen 4 kennzeichnet grob schematisch eine Umschalteinrichtung mittels derer von einer Beaufschlagung der Kunststoffvorformlinge mit dem ersten fließfähigen Medium (insbesondere Sterilluft) auf eine Beaufschlagung der Kunststoffvorformlinge mit dem zweiten fließfähigen Medium umgestellt werden kann.

Das Bezugszeichen 8 kennzeichnet ebenfalls einen Bestandteil der Zurverfügungstellungseinrichtung für das erste fließfähige Medium. Dieses kann beispielsweise eine Kompressoreinrichtung 16 aufweisen, welche Druckluft zur Verfügung stellt. Daneben kann auch eine lonisierungseinrichtung 18 vorgesehen sein, welche die Druckluft ionisiert, und welche auf diese Weise über (nicht gezeigte) Verbindungsleitungen den Beaufschlagungselementen 62 zugeführt werden kann.

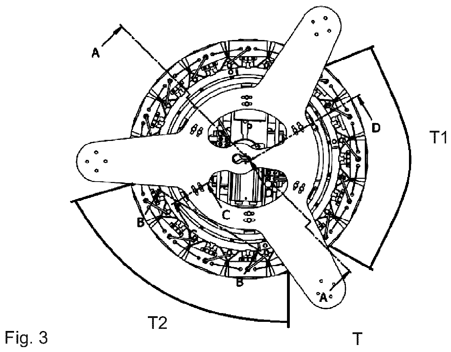

Fig. 3 zeigt eine Draufsicht auf die erfindungsgemäße Vorrichtung. Dabei kennzeichnet das Bezugszeichen T1 einen ersten Transportpfadabschnitt, entlang dessen die Kunststoffvorformlinge mit dem ersten fließfähigen Medium beaufschlagt werden und das Bezugszeichen T2 einen zweiten Transportpfadabschnitt, entlang dessen die Kunststoffvorformlinge mit dem zweiten fließfähigen Medium beaufschlagt werden. Der gesamte Transportpfad T der Kunststoffvorformlinge setzt sich hier aus den beiden Transportpfadabschnitten T1 und T2 zusammen.

## Patentansprüche

1. Vorrichtung zum Behandeln von Kunststoffvorformlingen (10) mit einer Transporteinrichtung (2), welche die Kunststoffvorformlinge entlang eines vorgegeben Transportpfads transportiert mit einer ersten Beaufschlagungseinrichtung (6), welche die Kunststoffvorformlinge (10) während deren Transport entlang eines ersten Transportpfadabschnitts (T1) durch deren Mündung (10a) hindurch mit einem ersten fließfähigen Medium beaufschlagt, und die Vorrichtung eine zweite Beaufschlagungseinrichtung (8) aufweist, welche die Kunststoffvorformlinge (10) während deren Transport entlang eines zweiten Transportpfadabschnitts (T2) durch deren Mündung (10a) hindurch mit einem zweiten fließfähigen Medium beaufschlagt und wenigstens eine Beaufschlagungseinrichtung (6, 8) eine lonisierungseinrichtung zum Ionisieren von gefilterter Luft aufweist,
**dadurch gekennzeichnet, dass**
die erste Beaufschlagungseinrichtung (6) und die zweite Beaufschlagungseinrichtung (8) ein Beaufschlagungselement (62) aufweisen, wobei die erste Beaufschlagungseinrichtung (6) und die zweite Beaufschlagungseinrichtung (8) das gleiche Beaufschlagungselement (62) zur Beaufschlagung der Kunststoffvorformlinge (10) mit dem ersten fließfähigen Medium und zur Beaufschlagung der Kunststoffvorformlinge mit dem zweiten fließfähigen Medium nutzen.

2. Vorrichtung nach Anspruch 1,
**dadurch gekennzeichnet, dass**
eine Beaufschlagungseinrichtung (6, 8) eine Zurverfügungstellungseinheit aufweist, welche Druckluft und insbesondere sterile Druckluft zur Verfügung stellt.

3. Vorrichtung (1) nach wenigstens einem der vorangegangenen Ansprüche,
**dadurch gekennzeichnet, dass**
wenigstens eine Beaufschlagungseinrichtung (6, 8) eine Zurverfügungstellungseinheit (62) aufweist, welche ein fließfähiges Sterilisationsmedium zur Verfügung stellt.

4. Vorrichtung (1) nach wenigstens einem der vorangegangenen Ansprüche,
**dadurch gekennzeichnet, dass**
die Vorrichtung (1) eine Absaugeinrichtung aufweist, um ein fließfähiges Medium aus dem Kunststoffvorformling abzusaugen.

5. Vorrichtung (1) nach dem vorangegangenen Anspruch,
**dadurch gekennzeichnet, dass**
die Vorrichtung eine Umschalteinrichtung (4) aufweist, welche ein Umschalten von einer Beaufschlagung der Kunststoffvorformlinge mit dem ersten fließfähigen Medium mittels der Beaufschlagungselemente zu einer Beaufschlagung der Kunststoffvorformlinge mit dem zweiten fließfähigen Mediums mittels der Beaufschlagungselemente umschaltet.

6. Vorrichtung (1) nach wenigstens einem der vorangegangenen Ansprüche,
**dadurch gekennzeichnet, dass**
die Transporteinrichtung (2) einen umlaufenden Träger (20) und insbesondere einen bezüglich einer Drehachse drehbaren Träger aufweist, an dem eine Vielzahl von Halteeinrichtungen (22) zum Halten der Kunststoffvorformlinge (10) angeordnet ist, wobei jeder Halteeinrichtung (22) wenigstens ein Beaufschlagungselement (62) zum Beaufschlagen der Kunststoffvorformlinge mit dem fließfähigen Medium zugeordnet ist.

7. Anordnung zum Behandeln von Kunststoffvorformlingen mit einer Vorrichtung nach wenigstens einem der vorangegangenen Ansprüche und einer in der Transportrichtung der Kunststoffvorformlinge der Vorrichtung nachgeordneten Vorrichtung (60) zum Sterilisieren von Kunststoffvorformlingen oder Kunststoffbehältnissen.

8. Verfahren zum Behandeln von Kunststoffvorformlingen (10) wobei eine Transporteinrichtung (2), die Kunststoffvorformlinge entlang eines vorgegeben Transportpfads transportiert mit einer ersten Beaufschlagungseinrichtung (6) welche die Kunststoffvorformlinge (10) während deren Transport entlang eines ersten Transportpfadabschnitts (T1) durch deren Mündung (10a) hindurch mit einem ersten fließfähigen Medium beaufschlagt, und eine zweite Beaufschlagungseinrichtung (8) die Kunststoffvorformlinge (10) während deren Transport entlang eines zweiten Transportpfadabschnitts (T2) durch deren Mündung (10a) hindurch mit einem zweiten fließfähigen Medium beaufschlagt und wenigstens eine Beaufschlagungseinrichtung (6, 8) eine lonisierungseinrichtung zum Ionisieren von gefilterter Luft aufweist,
**dadurch gekennzeichnet, dass**
die erste Beaufschlagungseinrichtung (6) und die zweite Beaufschlagungseinrichtung (8) ein Beaufschlagungselement (62) aufweisen, wobei die erste Beaufschlagungseinrichtung (6) und die zweite Beaufschlagungseinrichtung (8) das gleiche Beaufschlagungselement (62) zur Beaufschlagung der Kunststoffvorformlinge (10) mit dem ersten fließfähigen Medium und zur Beaufschlagung der Kunststoffvorformlinge mit dem zweiten fließfähigen Medium nutzen.

## Claims

1. Apparatus for treating plastic preforms (10), having a transport device (2) which transports the plastic preforms along a predetermined transport path, having a first application device (6) which acts upon the plastic preforms (10) with a first flowable medium through their mouth (10a) during their transport along a first transport path section (T1), and the apparatus has a second application device (8) which acts upon the plastic preforms (10) with a second flowable medium during their transport along a second transport path section (T2) through their mouth (10a), and at least one application device (6, 8) has an ionising device for ionising air, **characterised in that**
the first application device (6) and the second application device (8) have an application element (62), wherein the first application device (6) and the second application device (8) use the same application element (62) to act upon the plastic preforms (10) with the first flowable medium and to act upon the plastic preforms with the second flowable medium.

2. Apparatus according to claim 1,
**characterised in that**
one application device (6, 8) has a providing unit which provides compressed air and in particular sterile compressed air.

3. Apparatus (1) according to at least one of the preceding claims, **characterised in that**
at least one application device (6, 8) comprises a providing unit (62) which provides a flowable sterilization medium.

4. Apparatus (1) according to at least one of the preceding claims, **characterised in that**
the apparatus (1) has a suction device for sucking off a flowable medium from the plastic preform.

5. Apparatus (1) according to the preceding claim,
**characterised in that**
the apparatus has a switching device (4) which switches over from acting upon the plastic preforms with the first flowable medium by means of the application elements to acting upon the plastic preforms with the second flowable medium by means of the application elements.

6. Apparatus (1) according to at least one of the preceding claims, **characterised in that**
the transport device (2) has a circumferential carrier (20) and in particular a carrier which is rotatable with respect to an axis of rotation and on which a plurality of holding devices (22) for holding the plastic preforms (10) is arranged, wherein each holding device (22) being assigned at least one application element (62) for acting upon the plastic preforms with the flowable medium.

7. Arrangement for treating plastic preforms with an apparatus according to at least one of the preceding claims and an apparatus (60) for sterilising plastic preforms or plastic containers arranged downstream of the apparatus in the transport direction of the plastic preforms.

8. Method for treating plastic preforms (10), wherein a transport device (2) transports the plastic preforms along a predetermined transport path with a first application device (6) which acts upon the plastic preforms (10) during their transport along a first transport path section (T1) through their mouth (10a) with a first flowable medium, and a second application device (8) acts upon the plastic preforms (10) with a second flowable medium during their transport along a second transport path section (T2) through their mouth (10a), and at least one application device (6, 8) has an ionising device for ionising air,
**characterised in that**
the first application device (6) and the second application device (8) have an application element (62), wherein the first application device (6) and the second application device (8) use the same application element (62) to act upon the plastic preforms (10) with the first flowable medium and to act upon the plastic preforms with the second flowable medium.

## Revendications

1. Dispositif de traitement de préformes en matière plastique (10) avec un système de transport (2), lequel transporte les préformes en matière plastique le long d'un trajet de transport prédéfini, présentant un premier système de sollicitation (6), lequel soumet les préformes en matière plastique (10) pendant leur transport le long d'un premier tronçon de trajet de transport (T1) par leur embouchure (10a) de part en part à l'action d'un premier milieu pouvant s'écouler, et le dispositif présente un deuxième système de sollicitation (8), lequel soumet les préformes en matière plastique (10) pendant leur transport le long d'un deuxième tronçon de trajet de transport (T2) par leur embouchure (10a) de part en part à l'action d'un deuxième milieu pouvant s'écouler et au moins un système de sollicitation (6, 8) présentant un système d'ionisation pour ioniser de l'air filtré,
**caractérisé en ce que**
le premier système de sollicitation (6) et le deuxième système de sollicitation (8) présentent un élément de sollicitation (62), dans lequel le premier système de sollicitation (6) et le deuxième système de sollicitation (8) utilisent le même élément de sollicitation (62) pour soumettre les préformes en matière plastique (10) à l'action du premier milieu pouvant s'écouler et pour soumettre les préformes en matière plastique à l'action du deuxième milieu pouvant s'écouler.

2. Dispositif selon la revendication 1,
**caractérisé en ce que**
un système de sollicitation (6, 8) présente une unité de mise à disposition, laquelle met à disposition de l'air comprimé et en particulier de l'air comprimé stérile.

3. Dispositif (1) selon au moins l'une quelconque des revendications précédentes,
**caractérisé en ce que**
au moins un système de sollicitation (6, 8) présente une unité de mise à disposition (62), laquelle met à disposition un milieu de stérilisation pouvant s'écouler.

4. Dispositif (1) selon au moins l'une quelconque des revendications précédentes,
**caractérisé en ce que**
le dispositif (1) présente un système d'évacuation par aspiration pour évacuer par aspiration un milieu pouvant s'écouler hors de la préforme en matière plastique.

5. Dispositif (1) selon la revendication précédente,
**caractérisé en ce que**
le dispositif présente un système de commutation (4), lequel permet une commutation d'une sollicitation des préformes en matière plastique avec le premier milieu pouvant s'écouler au moyen des éléments de sollicitation à une sollicitation des préformes en matière plastique avec le deuxième milieu pouvant s'écouler au moyen des éléments de sollicitation.

6. Dispositif (1) selon au moins l'une quelconque des revendications précédentes,
**caractérisé en ce que**
le système de transport (2) présente un support (20) périphérique et en particulier un support pouvant tourner par rapport à un axe de rotation, sur lequel une pluralité de système de maintien (22) destinés à maintenir les préformes en matière plastique (10) est disposée, dans lequel au moins un élément de sollicitation (62) destiné à soumettre les préformes en matière plastique à l'action du milieu pouvant s'écouler est associé à chaque système de maintien (22).

7. Ensemble de traitement de préformes en matière plastique avec un dispositif selon au moins l'une quelconque des revendications précédentes, et un dispositif (60) disposé en aval du dispositif dans la direction de transport des préformes en matière plastique pour stériliser des préformes en matière plastique ou des récipients en matière plastique.

8. Procédé de traitement de préformes en matière plastique (10), dans lequel un système de transport (2) transporte les préformes en matière plastique le long d'un trajet de transport prédéfini, avec un premier système de sollicitation (6), lequel soumet les préformes en matière plastique (10) pendant leur transport le long d'un premier tronçon de trajet de transport (T1) par leur embouchure (10a) de part en part à l'action d'un premier milieu pouvant s'écouler, et un deuxième système de sollicitation (8) soumet les préformes en matière plastique (10) pendant leur transport le long d'un deuxième tronçon de trajet de transport (T2) par leur embouchure (10a) de part en part à l'action d'un deuxième milieu pouvant s'écouler et au moins un système de sollicitation (6, 8) présente un système d'ionisation pour ioniser de l'air filtré,
**caractérisé en ce que**
le premier système de sollicitation (6) et le deuxième système de sollicitation (8) présentent un élément de sollicitation (62), dans lequel le premier système de sollicitation (6) et le deuxième système de sollicitation (8) utilisent le même élément de sollicitation (62) destiné à soumettre les préformes en matière plastique (10) à l'action du premier milieu pouvant s'écouler et pour soumettre les préformes en matière plastique à l'action du deuxième milieu pouvant s'écouler.
